# EUROPEAN PATENT APPLICATION

(11) **EP 1 095 612 A1**
(43) Date of publication of application: **02.05.2001**
(21) Application number: 99310226.8
(22) Date of filing: 17.12.1999
(51) Int. Cl.: A61B 5/0428

(54) **Portable ECG apparatus and method**

(30) Priority: 29.10.1999 US 429623
(71) Applicant: Naqvi, Arif, Dr., Richmond Hill, Ontario L4B 3R6 (CA)
(72) Inventor: Naqvi, Arif, Dr., Richmond Hill, Ontario L4B 3R6 (CA)
(74) Representative: Davies, Gregory Mark

(57) **Abstract**

A portable ECG apparatus and method therefor is provided. The portable ECG apparatus (20) includes an electrode-garment (24) wearable by a patient and that readily locates a plurality of ECG electrodes (E1-E13) in appropriate locations on a patient A presently preferred electrode-garment is a vest (36) having thirteen electrodes. Output from the electrodes is connected to a diagnostic unit (32). The diagnostic unit is operable to analyze data generated by the electrodes and present a diagnosis that is understandable to a lay-person. In one specific embodiment, the diagnosis gives the patient either an alarm message, (i.e. the patient should go to the hospital), a warning message (i.e. re-administers the test), or a normal message (i.e. the patient's ECG is normal and no visit to the hospital is required.) By providing an initial diagnosis that is understandable to the patient, the time between noticing the initial symptoms of the heart-attack and arrival at the hospital for treatment can be reduced.

## Description

### Field of the Invention

The present invention relates generally to medical diagnostic equipment and more particularly relates to a portable ECG apparatus and method.

### Background of the Invention

Electrocardiogram ("ECG") apparatuses and methods are well known. Once a potential heart-attack victim arrives at the hospital, an ECG is the primary tool used to dianose the patient's condition. Hospital ECG equipment generally comprises ten electrodes which are affixed to various positions about the patient's torso. The electrodes then generate electrical signals that are representative of electrical activity in the patient's body. When properly placed, the electrodes will generate signals that reflect the activity of the patient's heart.

The electrodes are connected to a diagnostic device, such as a computer, which receives the signals from the electrodes and presents the data as a graph on an output device, typically a printer. As will be understood by those of skill in the art, the interpretation of the displayed graph requires training and skill on the part of the attending physician. It is also known to incorporate software into the computer diagnostic device that interprets the electrode signals and presents the result of the operation on the output device. Prior art interpretation software, however, does not lend itself to interpretation by the lay-person; a highly skilled medical professional, typically a physician, is required in order to read the results on the output device and diagnose the patient's condition. In general, hospital-based ECG equipment is complex and requires operation by skilled medical professionals.

Hospital-based ECG equipment is an important and useful tool in the hospital environment where advanced patient care is available, but it is not suitable for portable use, or use by a patient and/or the patient's family who are attempting to obtain a quick diagnosis as to the patient's condition. Typically, the average heart attack patient does not arrive at the hospital until more than two hours have elapsed since noticing initial symptoms, yet this is typically the time period when most of the damage occurs to the patient's heart.

In order to provide more portable ECG apparatuses, it is known to provide a garment, usually a strap, vest or belt, that has one or more ECG electrodes affixed thereon. A patient can simply wear the garment in order to quickly place the ECG electrodes. See, for example, U.S. Patent 4, 608,987 to Mills; U.S. Patent 4,709,704 to Lukasiewicz; U.S. Patent 3,534,727 to Roman; U.S. Patent 5,505,202 to Mogi; U.S. Patent 4,583,547 to Granek; U.S. Patent 5,788,633 to Mahoney. The primary teachings of these patents are directed to the actual garment, but they do not teach arrangements of electrodes suitable for an accurate, portable diagnosis. For example, prior art garments have ten or fewer electrodes, and as a result, can miss certain types of heart attacks. For example, quite commonly, the heart attack will affect the posterior or lateral wall of the heart, which can be poorly detected by ten-electrode ECG tests. Furthermore, prior art ECG apparatuses do not generally include portable diagnostic equipment that interprets the signals from the electrodes.

Mills teaches an apparatus including a vest and transmission equipment that transmits ECG to a remote location for diagnosis, thus allowing the patient to actually remain at home during the diagnosis. The Vest's electrodes are connected to a telephonic transmission unit, so that the signals from the vest can be interpreted by a diagnostic equipment that is remote to the vest. While the apparatus in Mills can be used by patients who are at home and suspect that they are having a heart-attack, the vest must still be connected to the telephonic transmission unit, and accordingly, the patient can still experience a delay between noticing heart-attack symptoms, fitting the vest, transmitting the ECG data, and waiting for a technician to respond as to the results of the diagnosis performed by the remote diagnostic equipment.

Heretofore diagnosis via ECGs are generally obtained using the analysis of a skilled physician, reviewing the output of a hospital based ECG machine that generates complex interpretations only readable by the physician. Overall, it can be seen that there is a need for a portable ECG apparatus and method that allows a potential heart-attack victim to readily obtain a preliminary diagnosis.

### SUMMARY OF THE INVENTION

It is therefore an object of the present invention to provide a novel portable ECG apparatus that obviates or mitigates at least one of the disadvantages of the prior art.

In one embodiment of the invention there is provided a portable ECG apparatus comprising an electrode-garment for affixing at leastone electrode to a patient's torso in a location for obtaining an ECG and a diagnostic unit having a microprocessor connectable to receive ECG data from the at least one electrode. The apparatus further includes a memory means for storing an operation that is executable by the microprocessor. During execution, theoperation can generate an alarm message when the ECG data indicates the patient is having a heart-attack.

In another embodiment of the invention there is provided a portable ECG apparatus comprising: an electrode-garment for affixing at least one electrode to a patient's torso in a location for obtaining an ECG and a diagnostic unit having a microprocessor connectable to receive ECG data from the at least one electrode. The diagnostic unit also has a memory means for storing an operation executable by the microprocessor, the operation for generating at least one of an alarm message, a warning message and a normal message based on whether the data indicates the patient is having a heart attack. The diagnostic unit also has a user-output device for presenting the generated message.

In another embodiment of the invention, there is provided a method for interpreting ECG electrode data comprising the steps of: receiving electrode data from at least one ECG electrode; generating a high-level ECG diagnosis from the at least one electrode; generating at least one of an alarm message, a warning message and a normal message from the high-level diagnosis; and presenting the at least one message to a person.
A portable ECG apparatus and method therefor is provided. The portable ECG apparatus includes an electrode-garment wearable by a patient and that readily locates a plurality of ECG electrodes in appropriate locations on a patient. A presently preferred electrode-garment is a vest having thirteen electrodes. Output from the electrodes is connected to a diagnostic unit. The diagnostic unit is operable to analyze data generated by the electrodes and present a diagnosis that is understandable to a lay-person In one specific embodiment, the diagnosis gives the patient at least one of an alarm. message, (i.e. the patient should go to the hospital), a warning message (i.e the test should be readministered), or a normal message (i.e. the patient's ECG is normal and no visit to the hospital is required.) By providing an initial diagnosis that is understandable to the patient, the time between noticing the initial symptoms of the heart-attack and arrival at the hospital for treatment can be reduced.

### BRIEF DESCRIPTION OF THE DRAWINGS

The present invention will now be explained, by way of example only, with reference to certain embodiments, and the attached Figures in which:
Figure 1 is a perspective view of a patient wearing a portable ECG apparatus in accordance with a first embodiment of the invention;
Figure 2 is a perspective view of the electrode-garment of Figure 1;
Figure 3 is an partial, interior perspective view of the electrode-garment of Figure 1;
Figure 4 is a front elevational view of the female-snap button of the electrodes view ofthe electrode-garment of Figure 3;
Figure 5 is a side elevational view of the female-snap button Figure 4;
Figure 6 is a front perspective view of the diagnostic unit of Figure 1;
Figure 7 is a front view of the diagnostic unit of Figure 6;
Figure 8 is a side view of the diagnostic unit of Figure 6;
Figure 9 is an end view of the diagnostic unit of Figure 6;
Figure 10 is a perspective view of the printed-circuit board of the unit of Figure 6;

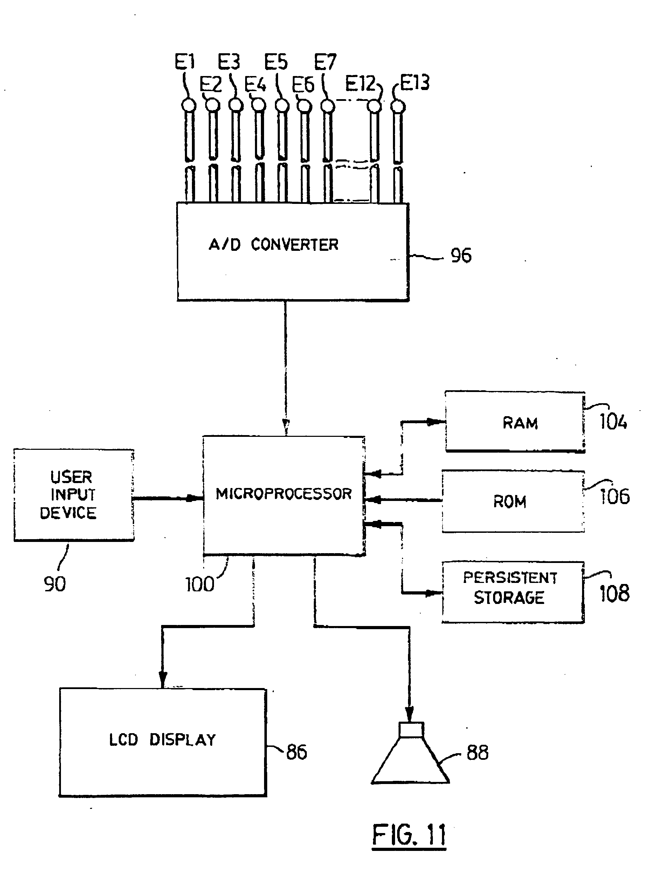

Figure 11 is a block-diagram of the components of the unit of Figure 6;

### DETAILED DESCRIPTION OF THE INVENTION

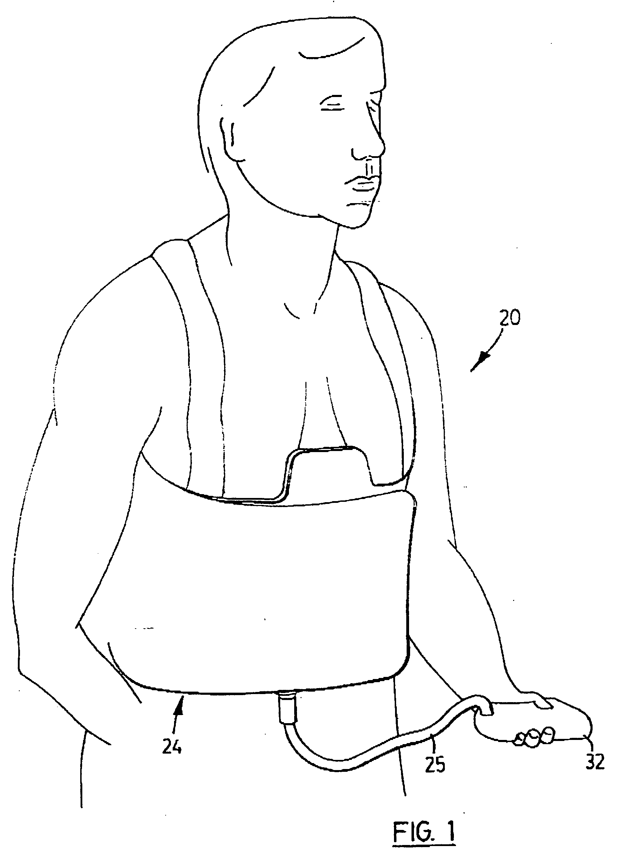

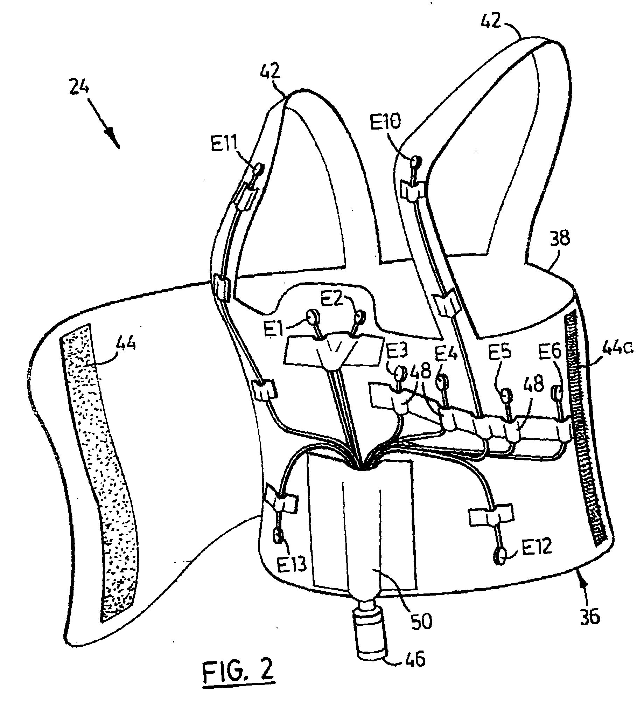

Referring now to Figure 1, a portable ECG apparatus in accordance with a first embodiment of the invention is indicated generally at 20. Apparatus 20 includes an electrode-garment 24, a cable 28 and a diagnostic unit 32. Referring nowto Figure 2, in a presently preferred embodiment electrode-garment 24 is a vest 36 having a plurality of electrodes E1-E13 attached at strategic locations throughout the vest Vest 36 has a torso portion 38 and a pair of shoulder-straps 42. Torso portion 38 is preferably made from a material that is comfortable on the skin, such as nylon fabric, however, other materials can be used. Torso portion 38 is a generally planar piece of fabric that can be wrapped about the torso of a person. A strip of Velcro^{TM} hooks 44 is attached to the free-end of torso portion 38 which correspond to a strip of Velcro^{TM} loops 44a on the mid-section of torso portion, to thus provide a means to affix the free-end of torso portion 38 about the person's torso. Attachment means other than books 44 and loops 44a will occur to those of skill in the art.

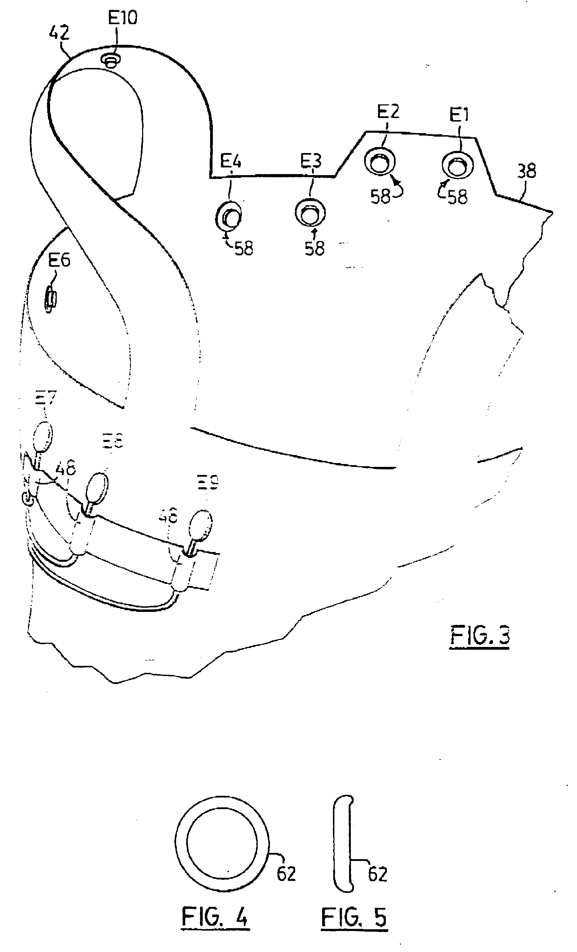

A main connector 46 is attached to each electrode E1-E13 via a wire respective to each electrodeE1-E13. The connection between each electrode E1-E13 and its respective wire is housed within a sleeve 48. A large sleeve 50 houses the bundle of wires leading to main connector 46. As best seen in Figure 3, electrodes E1-E13 are mounted through apertures 54 in torso portion 38. Each electrode E1-E13 has a male-snap button 58 which passes from the exterior of torso portion 38 and passes through its respective aperture 54 and projects into the interior of torso portion 38. As seen in Figures 4 and 5, each electrode E1-E13 also includes a female-snap button 62, which releasably engages its respective male-snap button 58. Each female-snap button 62 thus presents an electrically conductive surface that contacts the skin of the person when torso-portion 38 is affixed about the Person's torso. Each male-snap button 58 and female-snap button 62 is thus made from and/or coated with an electrically conductive material that has reduced susceptibility to corrosion, such as gold or silver. Table I lists the presently preferred electrode positions for each electrode E1-E13.

**TABLE I**

| **Electrode Number** | **Electrode Position** |
|---|---|
| E1 | Fourth intercostal space to the right of the sternum |
| E2 | Fourth intercostal space to the left of the sternum |
| E3 | Between E2 and E4 |
| E4 | Fifth intercostal space in the midclavicular line |
| E5 | Between E4 and E6 |
| E6 | Fifth intercostal space in the midaxillary line |
| E7 | Posterior axiallary line on the same level as E6 |
| E8 | On the back, beneath the angle of the left scapula |
| E9 | On the back, to the left of the vertebral column |
| E10 | Left shoulder |
| E11 | Right shoulder |
| E12 | Left lower abdomen |
| E13 | Right lower abdomen |

While the above configuration is presently preferred, it will be understood that other locations, numbers and/or configurations of electrodes can be used, as will occur to those of skill in the art.

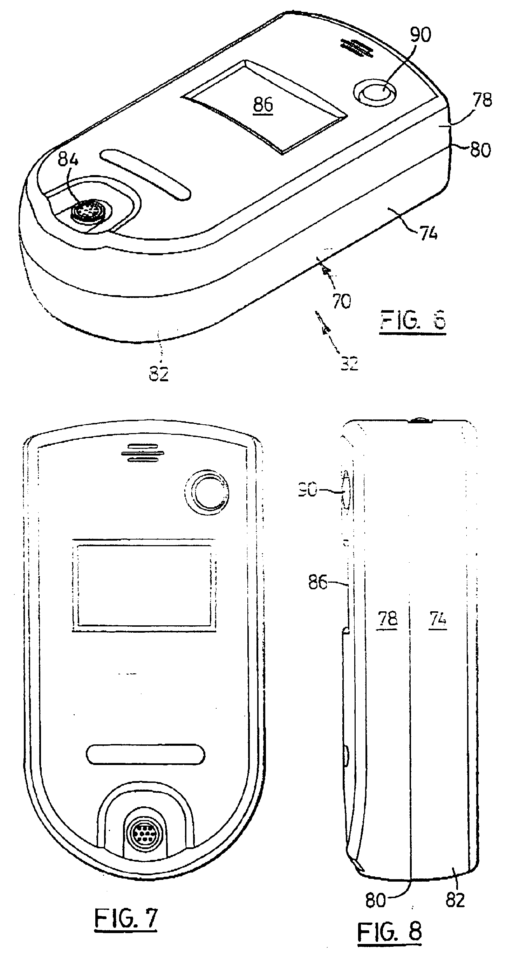

Accordingly, electrical signals detected by electrodes E1-E13 are transmitted via connector 26 and cable 28 to diagnostic unit 32. Referring now to Figures 6-9, diagnostic unit 32 has ahousing 70 that is preferably made of a durable plastic such as polypropylene, however, any other suitable material can be used. Housing 70 has a base portion 74 and a cover-portion 78, which join along a seam 80. Base portion has a lid 82 which allows access to a battery compartment for holding a power supply. Cover portion 78 houses an input connector 84 for connection with cable 28, an LCD display 86 for presenting messages to a user, a speaker 88, and a user-input device 90.

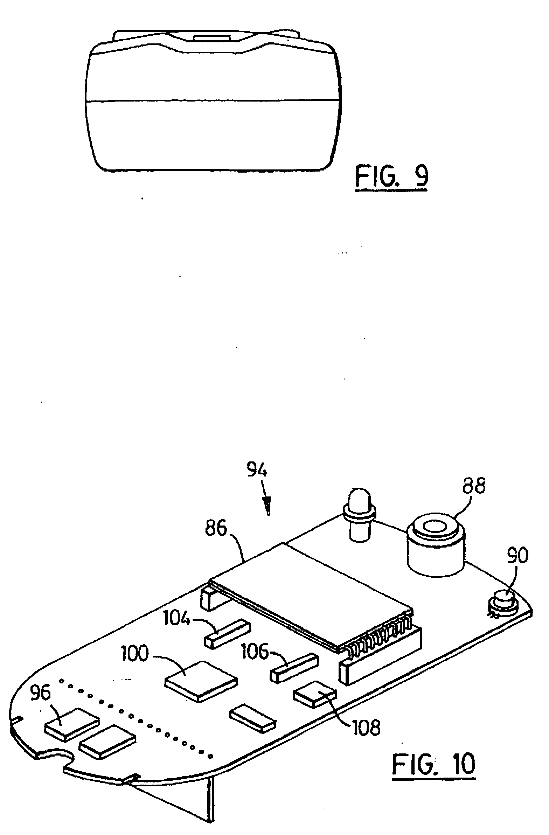

Referring now to Figure 10, a printed-circuit board 94 within diagnostic unit 32 is shown, including LCD display 86, speaker 88, and user-input device 90. Printed circuit board 94 further includes an analog-to-digital converter 96 which receives analog input from electrodes E1-E13 via connector 84 and converts the analog input into a digital signal that is sent to a microprocessor 100. Printed circuit board 94 further includes random access memory 104, read only memory 106 and persistent storage 108, which in a present embodiment is an EEPROM.

Referring now to Fig. 9, the interconnection of the elements on printed-circuit board 94 is shown as ablock diagram, whereby micro-processor 100 is operable to receive inputs from analog-to-digital converter 96 and user-input device 90. Microprocessor 100 is further operable to communicate with random access memory 104, read only memory 106 and persistent storage 108. Microprocessor 100 is further operable to present output to a uservia LCD display 86 and/orspeaker 88. The components shown in Figure 9 are powered via a battery, or other suitable power supply, the connection to which is not shown.

In a present embodiment, user-input device 90 is simply an on/off switch for cycling power to diagnostic unit 32, and accordingly, microprocessor 100 is operable to respond to such an input and accordingly cycle powerto LCD display 86, speaker 88 and the various other components within unit 32, as desired. RAM 104 is operable to communicate with microprocessor 100 and to store software executing on microprocessor 100 and/or temporary data required for such software. Persistent storage 108 is operable to record data collected from electrodes E1-E13 for further processing at a later date.

ROM 106 is operable to permanently store the software that is used by microprocessor 100 to analyze input signals from electrodes E1-E13. Any known high-level ECG analysis software can be used, such as "ST Ischaemia Analysis" from Zymed, 20 North Aviador Street, Camarillo, CA 93010. As known to those of skill in the art, there are number of such high-level ECG analysis software, and it should be understood that any such high-level ECG analysis software can be used, without departing from the scope of the invention.

As previously discussed, known high-level ECG analysis software produce highly complex outputs that are only suitable for interpretation by highly skilled medical professionals. Furthermore, such known ECG analysis software is used on hospital ECG equipment. Thus, in addition to the high-level ECG analysis software, ROM 106 further includes a low-level ECG analysis software that accesses a look-up table to correlate highly complex output from the high-level ECG analysis software to generate at least one of an alarm message, a warning message and a normal message and/or other diagnostic output that is comprehensible and/or usable by a lay-person. Table II shows an exemplary look-up table:

**TABLE II**

| **ECG Software Output for Physician** | **Interpretation for Lay-person** | **Type of message** |
|---|---|---|
| Heart rate > 140 | Heart rate too high-go to hospital! | Alarm |
| Heart rate < 40 | Heart rate too low-go to hospital! | Alarm |
| QRS duration >115 msec | Go to Hospital!! | Alarm |
| Q wave > 40 msec in any lead except III, AVR, V1 | Go to Hospital!! | Alarm |
| ST elevation > 1 mm in any lead | Go to Hospital!! | Alarm |
| ST depression > 0.5 mm in any lead | Go to Hospital!! | Alarm |
| T wave inversion in E3-E9 | Go to Hospital!! | Alarm |
| Abnormal T axis | Go to Hospital!! | Alarm |
| Poor Tracing | Readjust electrodes and sit still. | Warning |
| Cannot Read Electrode | Poor electrical connection - check leads. | Warning |
| All others | Normal - Repeat test in two minutes | Normal |

The exact contents of Table II can be varied and/or modified in accordance with the particular high-level ECG analysis software, as desired.

ROM 106 can further include an additional look-up table that compares a present reading from electrodes E1-E13, with one or more prior base-line readings kept in persistent storage 108. Table III shows an exemplary look-up table, where the left column shows the interpretation of the present reading with the stored reading in storage 108, and the right column shows the interpretation for the layperson user:

**TABLE III**

| **Detected variation from base-line** | **Interpretation for Layperson** | **Type of message** |
|---|---|---|
| QRS duration increased by > 15msec | Go to Hospital!! | Alarm |
| ST Segment Shift >0.5mm in any electrode | Go to Hospital!! | Alarm |
| T Wave axis shift | Go to Hospital!! | Alarm |
| New Q Waves | Go to Hospital!! | Alarm |
| No change from baseline | Normal - repeat test in two minutes. | Normal |

The operation of portable ECG apparatus 20 will now be discussed. Garment 24 is sized so that electrodes E1-E13 are appropriately positioned for a patient As soon as the patient experiences any symptoms that can indicate a-heart-attack, the patient adorns garment 24 by placing straps 42 over the appropriate shoulders. Electrodes E1-E13 are generally located over their appropriate positions on the patient, and torso-portion 38 is wrapped about the patient and hooks 44 are engaged with loops 46 to attach garment 24 to the patient.

Next, cable 28 is attached to connector 46 and input connector 84 to interconnect garment 24 and diagnostic unit 32. User-input device 90 is then actuated by depression, which sends a signal to microprocessor 100 to commence reading and interpretation. Next, microprocessor 100 receives digital input from electrodes E1-E13 via A/D converter 96. The digital input is then analyzed using known operations to arrive at a high-level interpretation suitable for analysis by a medical professional. Next, microprocessor 100 accesses Table II in ROM 106 in order to determine an interpretation suitable for presentation to a lay-person, such as the patient or a person assisting the patient. For purposes of explaining the operation, it will be assumed that the known operation determined that the patient's "QRS duration >115 msec", and, upon examination of Table II determines thatthe suitable interpretation is "Go to Hospital!!!" Next, microprocessor 100 transfers the interpretation "Go to Hospital!!!" to LCD Display 86 and/or to speaker 88 where the interpretation is announced. Atthis point, the operation is complete as the patient has been informed that their ECG reading is dangerous and that they require medical attention at a hospital.

It will now be apparent that where other interpretations are made, that the appropriate interpretation can be presented to the patient either via LCD Display 86, speaker 88 or other user-output device to assist in providing a diagnosis for the layperson. A simple user-output device could include a red, yellow and green LED that represent alarm, warning and normal messages, respectively.

While the embodiments discussed herein are directed to particular implementations of the present invention, itwill be apparent that the sub-sets and variations to these embodiments arewithin the scope of the invention. For example, it will be understood that other user-input devices 90, other than simply an on/off switch, can be added, such as a full keyboard for providing a variety of inputs to microprocessor l00. Further, other user-output devices can be added, in addition to LED display 86 and speaker 88, such as a serial cable link to a computer, or a modem. In turn, software stored on ROM 106 and executing on microprocessor 100 can be responsive to such inputs for providing a variety of outputs to the user, such as showing ahistory ofrecordings, clearing memory, uploading the contents of the memory to a computer and/or downloading new software for diagnostic unit 32.

It is also contemplated that the electrode- garment can be designed in a standard set of sizes, such as small, medium and large that will generally appropriately place each electrode for the majority of individuals. Alternatively, it is contemplated that the electrode- garment canbe custom designed and/or fitted for a specific individual.

The electrode-garment can include a means for manually placing each of the electrodes in a variety of customized positions, and such positions can be marked so that the electrodes can be changed so that one decode-garment can be shared by a number of individuals.

It is further contemplated that the electrode-garment can have a means to automatically control the placement of each electrode, such as through the use of a pair of servo-motors that move each electrode in plane. The motors can be connected to the micro-processor, and avariety of pre-set positions can be stored to accommodate appropriate electrode placement in avariety of different of individuals sharing the same garment.

It is contemplated that the software analysis of the present invention can be based on vectorcardiographic analysis of the ECG, that generates an appropriate message, such as an alarm, depending on the results of the analysis.

It will be understood that the look-up table can be eliminated, and that the diagnostic unit can simply contain software that analyzes the signals generated by the electrodes in the electrode-garment, and provides a message to the user, such as an alarm, a warning or a normal message.

The present invention provides a novel portable ECG apparatus that includes an electrode-garment connected to a diagnostic unit The diagnostic unit is operable to provide, to the lay-person, an interpretation of the ECG signal generated by a patient wearing the garment The provision of thirteen or greater electrodes in the electrode-garment can further improve the accuracy of the apparatus. Because a timely diagnosis of a heart-attack can be critical in treating the patient, and because many patients mis-diagnose their symptoms, as, for example, indigestion, the present invention allows a person at risk of a heart-attack or other coronary disease to obtain a diagnosis that is understandable to the lay-person and can thus decrease the time between the initial diagnosis of the heart-attack and the arrival at a hospital for treatment.

## Claims

1. A portable ECG apparatus comprising:
an electrode-garment for affixing at least one electrode to a patient's torso in a location for obtaining an ECG; and,
a diagnostic unithaving a microprocessor connectable to receiveECG data from said at least one electrode, said unit further having a memory means for storing an operation executable by said microprocessor, said operation for generating an alarm message when said data indicates said patient is having a heart-attack.

2. A portable ECG apparatus comprising:
an electrode-garment for affixing at least one electrode to a patient's torso in a location for obtaining an ECG; and
a diagnostic unit having amicroprocessor connecrable to receive ECG data from said at least one electrode, said unit further having a memory means for storing an operation executable by said microprocessor, said operation for generating at least one of an alarm message, a warning message and a normal message based on whether said data indicates said patient is having a heart attack, said unit further having a user-output device for presenting said generated message.

3. The portable ECG apparatus according to claim 2 wherein said electrode-garment is a vest having thirteen electrodes.

4. The portable ECG apparatus according to claim 3 wherein said thirteen electrodes are positioned, respectively, at the locations defined in the table below:
| **Electrode Number** | **Electrode Position** |
|---|---|
| E1 | Fourth intercostal space to the right of the sternum |
| E2 | Fourth intercostal space to the left of the sternum |
| E3 | Between E2 and E3 |
| E4 | Fifth intercostal space in the midclavicular line |
| E5 | Between E4 and E6 |
| E6 | Fifth intercostal space in the midaxillary line |
| E7 | Posterior axiallary line on the same level as E6 |
| E8 | On the back, beneath the angle of the left scapula |
| E9 | On the back, to the left of the vertebral column |
| E10 | Left shoulder |
| E11 | Right shoulder |
| E12 | Left lower abdomen |
| E13 | Right lower abdomen |

5. The portable ECG apparatus of claim 2 wherein said user-output device is an LCD screen.

6. The portable ECG apparatus of claim 2 wherein said user-output device includes a speaker.

7. The portable ECG apparatus of claim 2 wherein said operation includes high-level ECG analysis for generating a complex ECG diagnosis from said electrode data, said operation further including a low-level ECG analysis that generates said at least one message based on said complex ECG diagnosis.

8. The apparatus according to claim 2 wherein said microprocessor is further operable to store said ECG data in said memory means and generate said at least one message based on a comparison of said ECG data with subsequent ECG data received from said at least one electrode.

9. An ECG diagnostic unit comprising:
a connector for attachment to at least one ECG electrode, said diagnostic unit further having a microprocessor connectable to receive ECG data from said at least one electrode, said unit further having a memory means for storing an operation executable by said microprocessor that considers said data to generate at least one of an alarm message, a warning message and a normal message, said unit further having a user-output device for presenting said generated message.

10. A vest for affixing thirteen electrodes to a patient's torso in locations for obtaining a ECG data, said electrode-garment further including a connector for attachment to a diagnostic unit considers said data to generate at least one of an alarm message, awarning message and a normal message, said unit further having a user-output device for presenting said generated message.

11. A method for interpreting ECG electrode data comprising the steps of:
receiving electrode data from at least one ECG electrode;
generating a high-level ECG diagnosis from said at least one electrode;
generating at least one of an alarm message, a warning message and a normal message from said high-level diagnosis; and
presenting said at least one message to a person.

12. The method according to claim 11 further comprising the step of receiving subsequent electrode data from said at least one ECG electrode and wherein said step of generating said at least one message is based on a comparison of said subsequent electrode data with said electrode data.
